# EUROPÄISCHE PATENTSCHRIFT

(11) **EP 0 569 778 B1**
(45) Veröffentlichungstag und Bekanntmachung des Hinweises auf die Patenterteilung: **10.01.1996**
(21) Anmeldenummer: 93106885.2
(22) Anmeldetag: 28.04.1993
(51) Int. Cl.: C07C 409/38

(54) **Oberflächenaktive Peroxide und ihre Verwendung**
Surface-active peroxides and their use
Péroxydes tensio-actifs et leur utilisation

(30) Priorität: 11.05.1992 DE 4215484
(43) Veröffentlichungstag der Anmeldung: 18.11.1993
(73) Patentinhaber: BAYER AG, D-51368 Leverkusen (DE)
(72) Erfinder: Bohnenpoll, Martin, Dr., W-5000 Köln 80 (DE); Schmidt, Adolf, Dr., W-5000 Köln 80 (DE); Alberts, Heinrich, Dr., W-5068 Odenthal (DE)

(56) Entgegenhaltungen:
- FR-A- 2 007 985
- US-A- 2 567 615
- US-A- 3 082 236
- CHEMICAL ABSTRACTS, vol. 115, 1991, Columbus, Ohio, US; abstract no. 161232k, CALLAIS, P.A. 'The synthesis of acrylic HSC resins with organic peroxides containing a hydroxy or carboxylic acid functional group.' Seite 106 ;Spalte 2 ;
- CHEMICAL ABSTRACTS, vol. 96, 1982, Columbus, Ohio, US; abstract no. 103592f, FEDOROVA, V.A. ET AL. 'Synthesis of acid monoper esters of aliphatic dibasic acids.' Seite 648 ;Spalte 2 ;
- CHEMICAL ABSTRACTS, vol. 98, 1983, Columbus, Ohio, US; abstract no. 106766t, KOVBUZ, M.A. ET AL. 'Synthesis of (O,O'-dicarbotert-butylperoxy) dicarboxylic acid peroxides.' Seite 555 ;Spalte 2 ;
- CHEMICAL ABSTRACTS, vol. 107, 1987, Columbus, Ohio, US; abstract no. 40405v, NAKASHIO, YUKIYASU ET AL. 'Synthesis of new peroxy esters and the ability of initiation for polymerization of styrene.' Seite 5 ;Spalte 1 ;
- CHEMICAL ABSTRACTS, vol. 93, 1980, Columbus, Ohio, US; abstract no. 8630w, LADOUSSE A. ET AL. 'Functional radical initiators. 1. tert.-Butyldioxycarbonylbenzoic acids.' Seite 8 ;Spalte 1 ;
- CHEMICAL ABSTRACTS, vol. 84, 1976, Columbus, Ohio, US; abstract no. 30475k, HIROTOMI, OSAMU 'Aliphatic dicarboxylic acid monoperoxy esters.' Seite 410 ;Spalte 1 ;
- CHEMICAL ABSTRACTS, vol. 106, 1987, Columbus, Ohio, US; abstract no. 67838e, SAWADA,HIDEO 'Carboxy-containing peroxides as polymerization initiators' Seite 12 ;Spalte 2 ;
- CHEMICAL ABSTRACTS, vol. 87, 1977, Columbus, Ohio, US; abstract no. 167672d, DONCHAK, V.A. ET AL. 'Peroxide esters, derivatives of pyromellitic and trimellitic acids.' Seite 543 ;Spalte 2 ;

## Beschreibung

Die vorliegende Erfindung betrifft oberflächenaktive Peroxide, die als emulgierend wirkende Initiatoren zur Herstellung von elektrolytarmen Polymerisat-Dispersionen mit geringer Tendenz zur Schaumbildung eingesetzt werden können.

Bei der Emulsionspolymerisation besteht das Problem, daß nach dem Ausfällen des gebildeten Polymers ein stark schäumendes Abwasser zurückbleibt, das aus Gründen des Umweltschutzes unter hohen Kosten entsorgt werden muß. Ziel der Erfindung war die Entwicklung von Emulgatoren, die bei der Polymerisation in das entstehende Produkt eingebaut werden und deshalb das Abwasser nicht mehr belasten können.

Es ist bekannt, zur Lösung dieses Problems Azoinitiatoren mit oberflächenaktiven Funktionalitäten zur Herstellung derartiger Dispersionen einzusetzen (vgl. EP-A 65 661, EP-A 65 136).

Diese Initiatoren eignen sich zwar zur Herstellung von schaumarmen Latices, sind jedoch wegen umständlicher Synthesen im technischen Maßstab schlecht zugänglich.

Eine Verbesserung bieten Peroxide mit oberflächenaktiven funktionellen Gruppen. Verbindungen mit einer geeigneten Struktur sind im Prinzip bekannt. Das japanische Patent JP 61 192 704 beschreibt Peroxidinitiatoren mit bis zu 18 C-Atomen, die zur Polymerisation in Lösung oder in Substanz eingesetzt werden können. Der Einsatz dieser Verbindungen in der Emulsionspolymerisation ist jedoch noch nicht beschrieben. Hinzu kommt, daß zur Erzielung einer guten Emulgatorwirkung bevorzugt Verbindungen eingesetzt werden, die über mehr C-Atome verfügen, als die in diesem Patent beschriebenen Verbindungen.

Aus FR 2 007 985 sind Peroxidinitiatoren für die Emulsionspolymerisation beschrieben, die in ihren emulgierenden Eigenschaften noch gewisse Nachteile aufweisen.

Aus US-A 2.567.615 ist ein Verfahren zur Herstellung von Alkyl-Perestern bekannt, bei dem ein tert.-Alkylhydroperoxid und ein organisches Säurechlorid, -fluorid oder -anhydrid miteinander reagieren.

In Chemical Abstract Vol. 93, 1980, No 8630w wird die Kinetik der Thermolyse von speziellen tert.-Butyl-per-phthalsäuren untersucht.

Erfindungsgemäß werden leicht zu synthetisierende Initiator-Emulgator-System bereitgestellt, mit denen Dispersionen mit geringer Tendenz zur Schaumbildung und geringem Elektrolytgehalt hergestellt werden können. Es handelt sich dabei um teilweise noch neue oberflächenaktive Peroxidverbindungen mit eingebauten Carbonsäure- oder Sulfonsäuregruppen und deren Salze, die gleichzeitig emulgierende und aktivierende Eigenschaften besitzen. Diese wirken zunächst als Emulgatoren und zerfallen während der Polymerisation. Die Bruchstücke werden in das Polymer eingebaut und schützen die Latexteilchen vor Koagulation.

Gegenstand der Erfindung ist die Verwendung oberflächenaktiver Peroxidverbindungen der allgemeinen Formel (I) 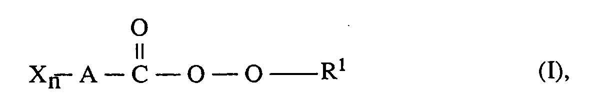
worin bedeuten
- R¹: Alkyl-, Cycloalkyl- und Arylreste mit 4 bis 20 Kohlenstoffatomen,
- X: die Reste -COOH und -SO₃H sowie deren Alkali-, Erdalkali- oder Ammoniumsalze,
- n: eine ganze Zahl im Bereich von 1 bis 6; n steht bevorzugt für die Zahlen 1 und 2,
- A: einen Alkylenrest mit 6 bis 40, vorzugsweise 10 bis 17, Kohlenstoffatomen.
zur Emulsionspolymerisation olefinisch ungesättigter Monomerer.

Bevorzugt steht der Rest A für einen Rest der Formel (II) 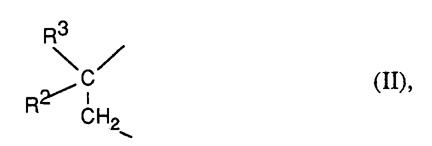
worin
die Reste R² und R³ für H, C₁-C₄₀-Alkyl, C₃-C₄₀-Cycloalkyl, Aryl, Aralkyl oder Halogen stehen, mit der Einschränkung, daß die Summe der Kohlenstoffatome beider Reste größer als 16 ist. Weiterhin können die Reste Bestandteil eines C₅-C₁₀ aliphatischen oder C₆-C₁₄ aromatischen Ringsystems sein.

Die erfindungsgemäß verwendeten Peroxide haben emulgierende Eigenschaften und können bei der Emulsionspolymerisation olefinisch ungesättigter Monomerer als Initiatoren und Emulgatoren zur Herstellung schaumarmer Latices eingesetzt werden.

Die erfindungsgemäß verwendeten Verbindungen sind Umsetzungsprodukte von Hydroperoxiden mit cyclischen Säureanhydriden.

Beispiele für geeignete Hydroperoxide sind tert.-Butylhydroperoxid, tert.-Amylhydroperoxid, Cumolhydroperoxid und Pinanhydroperoxid.

Cyclische Säureanhydride im Sinne der Erfindung sind cyclische Anhydride von Carbon- oder Sulfonsäuren mit bis zu 40 Kohlenstoffatomen, bevorzugt mit 21 bis 40 Kohlenstoffatomen. Sie können halogeniert oder nitriert sein.

Beispiele für geeignete cyclische Säureanhydride sind:
N-Octadecylbernsteinsäureanhydrid, N-Octadecenylbernsteinsäureanhydrid, Isooctadecenylbernsteinsäureanhydrid, Isooctadecylbernsteinsäureanhydrid, 2-Octadecenylbernsteinsäureanhydrid, N-Eicosylbernsteinsäureanhydrid, N-Docosenylbernsteinsäureanhydrid, N-Docosylbernsteinsäureanhydrid oder Mischungen daraus.

Diese Substanzen können sowohl in reiner Form, als auch als technische Gemische eingesetzt werden. Bevorzugt werden Gemische, die dadurch gekennzeichnet sind, daß sie bei Raumtemperatur flüssig vorliegen.

Die Herstellung der erfindungsgemäß verwendeten Verbindungen ist nach der folgenden allgemeinen Methode möglich:
Das gewählte Hydroperoxid wird in einem geeigneten Solvens gelöst und mit einem cyclischen Säureanhydrid umgesetzt.

Als Solvens für die Reaktion sind Wasser, organische Lösungsmittel und Zweiphasensysteme aus Wasser und einem mit Wasser nicht lösbaren organischen Lösungsmittel geeignet.

Verwendbare organische Lösungsmittel sind solche, die unter den Reaktionsbedingungen inert sind, ein ausreichendes Lösungsvermögen für die Ausgangsmaterialien und das entstehende Produkt besitzen und sich bei Temperaturen von höchstens 50°C im Vakuum destillieren lassen.

Als Beispiele für mögliche organische Lösungsmittel seien genannt: Benzol, Toluol, Methylenchlorid, Chloroform, Essigsäuremethylester, Essigsäureethylester, Essigsäurepropylester, Essigsäurebutylester und Acetonitril.

Bevorzugte Lösungsmittel sind Wasser, Methylenchlorid, Toluol und ein Zweiphasensystem aus Wasser und Methylenchlorid.

Besonders bevorzugt wird die Herstellung der erfindungsgemäßen Peroxide in Wasser.

Die Reaktionstemperatur kann prinzipiell in einem weiten Rahmen variiert werden. So sind z.B. Temperaturen im Bereich von 0 bis 50°C möglich. Bevorzugt wird jedoch bei Temperaturen im Bereich von 15 bis 25°C gearbeitet.

Die Reaktionsdauer ist variabel und hängt im wesentlichen von der Reaktionstemperatur und der Reaktivität der einzelnen Verbindungen ab und liegt üblicherweise im Bereich von 0,5 bis 40 Stunden. Bevorzugt wird eine Reaktionsdauer im Bereich von 1 bis 30 Stunden, besonders bevorzugt im Bereich von 5 bis 20 Stunden.

Die Dosierung des Säureanhydrids, bezogen auf das eingesetzte Hydroperoxid, beträgt 0,9 bis 1,1 Mol, bevorzugt 0,95 bis 1,05 Mol.

Falls gewünscht, kann eine Aufarbeitung durch Eindampfen des Lösungsmittels erfolgen, gegebenenfalls im Hochvakuum bei Temperaturen bis zu 50°C. Bevorzugt werden die Verbindungen jedoch nicht vollständig getrocknet, sondern als Lösungen weiterverwendet. Der Gehalt der Lösungen kann in einem sehr weiteren Bereich variieren und zwischen 1 und 90 % liegen.

Der pH-Wert der Lösungen kann durch Zugabe von Säuren oder Basen auf einen gewünschten Wert eingestellt werden.

Bevorzugt ist die Verwendung der Peroxidverbindungen der Formel (I), sowie von Verbindungen, in denen der Rest A für Radikale mit 8 bis 17 C-Atomen steht, bei der Emulsionspolymerisation eines oder mehrerer olefinisch ungesättigter Monomerer zur Herstellung von elektrolytarmen Dispersionen mit geringer Tendenz zur Schaumbildung. Diese Verbindungen werden in Mengen von 0,05 bis 10 Gew.-%, vorzugsweise 0,1 bis 6 Gew.-%, besonders bevorzugt 1 bis 3 Gew.-%, bezogen auf Monomere, eingesetzt.

Die Peroxidverbindungen können entweder als fertige Verbindungen in die Polymerisation eingesetzt werden, oder unmittelbar vor Beginn der Polymerisationsreaktion in situ erzeugt werden.

Die Emulsionspolymerisation mit Hilfe der neuen Peroxidemulgatoren kann mit diesen Emulgatoren allein oder auch in Kombination mit anionischen oder nicht-ionogenen Tensiden nach den bekannten Techniken der Emulsionspolymerisation absatzweise, halbkontinuierlich mit Monomerenzulauf, halbkontinuierlich mit Emulsionszulauf sowie kontinuierlich durchgeführt werden.

Bei allen erwähnten Polymerisationstechniken kann man sich auch vorgelegter oder während der Reaktion zugegebener Saatlatices bedienen, um die Teilchengrößenverteilung zu beeinflussen.

Man kann auch vorgelegte Saatlatices mit Monomeren in Gegenwart der erfindungsgemäß verwendeten Peroxidemulgatoren anquellen; dies gegebenenfalls bis zum Erreichen eines thermodynamischen Gleichgewichts, was Stunden bis mehrere Tage dauern kann und was bei Temperaturen erfolgt, welche unterhalb der Zersetzungstemperatur des Peroxidinitiators liegen. Danach kann man die Polymerisation durch Erwärmen auslösen.

Wenn man an der Oberfläche der Latexteilchen die anionischen, aus dem Peroxidinitiator stammenden Gruppen konzentrieren will, polymerisiert man mit Hilfe möglichst geringer Mengen niedermolekularer, gut dialysierbarer Emulgatoren in Gegenwart geringer Mengen der erfindungsgemäß verwendeten Peroxidemulgatoren, erhöht gegen Ende der Polymerisation die Peroxidemulgatordosierung und reduziert gleichzeitig den üblichen Emulgator. Die erfindungsgemäß verwendeten Peroxidemulgatoren beeinflussen dabei simultan das Molekulargewicht der sich bildenden Polymerketten sowie deren Bestückung mit hydrophilen Gruppen.

Entsprechend der Zerfallskinetik der Peroxidverbindungen wird die Polymerisation bei Temperaturen zwischen 35°C bis 90°C durchgeführt, bevorzugt bei 45°C bis 75°C.

Als Monomere kommen alle mit Peroxiden polymerisierbaren, olefinisch ungesättigten Monomeren in Betracht, beispielsweise Styrol, α-Methylstyrol, Butadien, C₁-C₈-Alkylacrylate, C₁-C₈-Alkylmethacrylate, Acrylnitril, Methacrylnitril, Vinylchlorid, Vinylacetat, Ethylen, Chloropren und deren Mischungen.

Zusätzlich können in geringeren Mengen wasserlösliche Verbindungen wie Methacrylsäure, Acrylsäure, Maleinsäurehalbester, Itaconsäure und Itaconsäurehalbester, Acrylamid, Methacrylamid usw. einpolymerisiert werden. Ferner können noch funktionelle Gruppen, z.B. OH- oder Epoxidgruppen tragende Comonomere mitverwendet werden, wie β-Hydroxyethyl(meth)acrylat, β-Hydroxy-propyl(meth)acrylat, Glycidyl(meth)acrylat und N-Methylol- bzw. N-Methylolalkylether des (Meth)Acrylsäureamids.

### Beispiele

Serienpolymerisationsversuche wurden in 500 ml fassenden verkorkten Glasflaschen mit zusätzlichem Kronkorkenverschluß (vgl. Houben-Weyl, Methoden der Organischen Chemie, Band 14/1, (1961), Seite 147) unter Ausschluß von Luftsauerstoff durchgeführt. Die zum Splitterschutz in Edelstahlkörbe eingesetzten Glasflaschen rotierten bei einer Drehzahl von 25 Umdrehungen pro Minute. Die Temperatur des Wasserbades wurde konstant gehalten. Die Korken der Flaschen wurden vor ihrer Entnahme aus den Stahlkörben mittels Stahlkanüle durchbohrt, um einen vorhandenen Überdruck zu entspannen.

Die Emulgatoren wurden als 1 %ige wässrige Lösung zugesetzt.

Zur Messung der Schaumrückbildungszeit wurde eine Probe von 100 ml Latex 30 Sekungen lang kräftig geschüttelt. Anschließend wurde die Zeit bis zum Verschwinden des Schaums gemessen.

Die in den Beispielen und Vergleichsversuchen angegebenen Prozente und Teile beziehen sich auf das Gewicht, sofern nicht anders vermerkt.

Bezüglich der Messung der Latexteilchendurchmesser wird auf die Fachliteratur (H.G. Müller, Colloid. Polym. Sci. 267, 1113-1116 (1989 und die dort zitierte Quellenliteratur) verwiesen.

### Herstellung der Emulgatoren

### Beispiel 1

0,72 g (0,003 Mol) Decylbernsteinsäureanhydrid wird in 20 ml Methylenchlorid und nach Zugabe von 0,57 g (0,003 Mol) Cumolhydroperoxid (80 %ig) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Suspension wird mit Wasser auf 25,8 g aufgefüllt.

### Beispiel 2

0,97 g (0,003 Mol) Hexadecylbernsteinsäureanhydrid wird in 20 ml Methylenchlorid und nach Zugabe von 0,57 g (0,003 Mol) Cumolhydroperoxid (80 %ig) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert Die zurückbleibende farblose Suspension wird mit Wasser auf 30,8 g aufgefüllt.

### Beispiel 3

0,89 g (0,003 Mol) Tetradecylbernsteinsäureanhydrid wird in 20 ml Methylenchlorid und nach Zugabe von 0,57 g (0,003 Mol) Cumolhydroperoxid (80 %ig) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Suspension wird mit Wasser auf 29,2 g aufgefüllt.

### Beispiel 4

1,06 g (0,003 Mol) Octadecylbernsteinsäureanhydrid wird in 20 ml Methylenchlorid und nach Zugabe von 0,57 g (0,003 Mol) Cumolhydroperoxid (80 %ig) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Suspension wird mit Wasser auf 32,6 g aufgefüllt.

### Beispiel 5

0,89 g (0,003 Mol) Tetradecylbernsteinsäureanhydrid wird in 20 ml Methylenchlorid und nach Zugabe von 0,38 g (0,003 Mol) tert.-Butylhydroperoxid (70 %ig in Wasser) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 23,2 g aufgefüllt.

### Beispiel 6

0,97 g (0,003 Mol) Hexadecylbernsteinsäureanhydrid wird in 20 ml Methylenchlorid und nach Zugabe von 0,38 g (0,003 Mol) tert.-Butylhydroperoxid (70 %ig in Wasser) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 24,8 g aufgefüllt.

### Beispiel 7

1,06 g (0,003 Mol) Octadecylbernsteinsäureanhydrid wird in 20 ml Methylenchlorid und nach Zugabe von 0,38 g (0,003 Mol) tert.-Butylhydroperoxid (70 %ig in Wasser) 4 Stunden bei Raumtemperatur gerührt. Anschließend wird eine Lösung von 0,12 g (0,003 Mol) Natriumhydroxid in 20 ml Wasser zugetropft und über Nacht bei Raumtemperatur gerührt. Durch Anlegen von Vakuum wird das Methylenchlorid abdestilliert Die zurückbleibende farblose Flüssigkeit wird mit Wasser auf 26,6 g aufgefüllt.

### Beispiel 8

0,72 g (0,003 Mol) Decylbernsteinsäureanhydrid wird in 20 ml Methylenchlorid und nach Zugabe von 0,38 g (0,003 Mol) tert.-Butylhydroperoxid (70 %ig in Wasser) 4 Stunden bei Raumtemperatur gerührt. Anschließend wird eine Lösung von 0,12 g (0,003 Mol) Natriumhydroxid in 15 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 19,8 g aufgefüllt.

### Beispiel 9

0,92 g (0,005 Mol) 2-Sulfobenzoesäureanhydrid wird in 20 ml Methylenchlorid und nach Zugabe von 0,64 g (0,005 Mol) tert.-Butylhydroperoxid (70 %ig in Wasser) 4 Stunden bei Raumtemperatur gerührt. Anschließend wird eine Lösung von 0,2 g (0,005 Mol) Natriumhydroxid in 15 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 27,4 g aufgefüllt.

### Beispiel 10

0,77 g (0,005 Mol) Hexahydrophthalsäureanhydrid wird in 20 ml Methylenchlorid und nach Zugabe von 0,64 g (0,005 Mol) tert.-Butylhydroperoxid (70 %ig in Wasser) 4 Stunden bei Raumtemperatur gerührt. Anschließend wird eine Lösung von 0,2 g (0,005 Mol) Natriumhydroxid in 15 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 24,4 g aufgefüllt.

### Beispiel 11

0,77 g (0,005 Mol) Hexahydrophthalsäureanhydrid wird in 20 ml Methylenchlorid und nach Zugabe von 0,95 g (0,005 Mol) Cumolhydroperoxid (80 %ig) 4 Stunden bei Raumtemperatur gerührt. Anschließend wird eine Lösung von 0,2 g (0,005 Mol) Natriumhydroxid in 20 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 30,6 g aufgefüllt.

### Beispiel 12

0,5 g (0,005 Mol) Bernsteinsäureanhydrid wird in 20 ml Methylenchlorid und nach Zugabe von 0,64 g (0,005 Mol) tert.-Butylhydroperoxid (70 %ig in Wasser) 4 Stunden bei Raumtemperatur gerührt. Anschließend wird eine Lösung von 0,2 g (0,005 Mol) Natriumhydroxid in 15 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 19 g aufgefüllt.

### Beispiel 13

0,5 g (0,005 Mol) Bernsteinsäureanhydrid wird in 20 ml Methylenchlorid und nach Zugabe von 0,95 g (0,005 Mol) Cumolhydroperoxid (80 %ig) 4 Stunden bei Raumtemperatur gerührt. Anschließend wird eine Lösung von 0,2 g (0,005 Mol) Natriumhydroxid in 15 ml Wasser zugetropft und über Nacht bei Raumtemperatur gerührt. Durch Anlegen von Vakuum wird das Methylenchlorid abdestilliert. Die zurückbleibende farblose Flüssigkeit wird mit Wasser auf 25,2 g aufgefüllt.

### Beispiel 14

0,112 g (0,002 Mol) KOH, gelöst in 20 ml Wasser, 0,256 g (0,002 Mol) tert.-Butylhydroperoxid (70 %ig) und 0,7 g (0,002 Mol) Octadecenylbernsteinsäure anhydrid werden 20 Stunden bei Raumtemperatur kräftig gerührt. Man erhält eine farblose, leicht trübe Flüssigkeit.

### Beispiel 15

0,224 g (0,004 Mol) KOH, gelöst in 40 ml Wasser, 0,76 g (0,004 Mol) Cumolhydroperoxid (80 %ig) und 1,4 g (0,004 Mol) Octadecenylbernsteinsäureanhydrid werden 20 Stunden bei Raumtemperatur kräftig gerührt. Man erhält eine farblose milchige Flüssigkeit

### Beispiel 16

0,224 g (0,004 Mol) KOH, gelöst in 40 ml Wasser, 0,76 g (0,004 Mol) Cumolhydroperoxid (80 %ig) und 1,41 g (0,004 Mol) Octadecylbernsteinsäureanhydrid werden 40 Stunden bei Raumtemperatur kräftig gerührt. Man erhält eine farblose, leicht trübe Flüssigkeit.

### Beispiel 17: Polymerisationsversuche mit Styrol

| | A | B | C | D | E | F |
|---|---|---|---|---|---|---|
| E-Wasser. ausgek.[g] | 153,35 | 136,69 | 120,040 | 153,65 | 137,30 | 120,96 |
| Natronlauge 0,1 n [ml] | 1,81 | 3,62 | 5,430 | 1,50 | 3,01 | 4,51 |
| Styrol [g] | 30,00 | 30,00 | 30,000 | 30,00 | 30,00 | 30,00 |
| Beispiel 1 (1 %ig) [g] | 15,00 | 30,00 | 45,000 | | | |
| Beispiel 2 (1 %ig) [g] | | | | 15,00 | 30,00 | 45,00 |
| Polym.-Dauer [Std.] | 15,00 | 15,00 | 15,000 | 15,00 | 15,00 | 15,00 |
| Polym.-Temp. [°C] | 70,00 | 70,00 | 70,000 | 70,00 | 70,00 | 70,00 |
| B [g] | 566,67 | 565,11 | 566,420 | 569,83 | 570,72 | 568,08 |
| T [g] | 367,71 | 365,66 | 366,950 | 370,82 | 371,55 | 368,76 |
| Ausfall, getr. [g] | 1,30 | 2,30 | 0,005 | 1,30 | 1,30 | 0,58 |
| Feststoff [%] | 4,54 | 10,87 | 12,980 | 13,37 | 9,78 | 13,20 |
| pH-Wert | 7,00 | 7,15 | 7,000 | 8,40 | 8,40 | 8,50 |
| el. Leitfähigk. [mS] | 0,36 | 0,33 | 0,460 | 0,09 | 0,13 | 0,17 |
| Photometr. Messung | | | | | | |
| Wl=500 nm [nm] | 424,00 | 393.00 | 315,000 | 359,00 | | |
| Wl=600 nm [nm] | 384,00 | 362,00 | 301,000 | 336,00 | | |
| Wl=700 nm [nm] | 369,00 | 353,00 | 299,000 | 331,00 | | |

### Beispiel 18: Polymerisationsversuche mit Styrol

| | A | B | C | D | E | F |
|---|---|---|---|---|---|---|
| E-Wasser. ausgek.[g] | 153,56 | 137,13 | 120,69 | 153,73 | 137,46 | 121,20 |
| Natronlauge 0,1 n [ml] | 1,59 | 3,19 | 4,78 | 1,42 | 2,85 | 4,27 |
| Styrol [g] | 30,00 | 30,00 | 30,00 | 30,00 | 30,00 | 30,00 |
| Beispiel 3 (1 %ig) [g] | 15,00 | 30,00 | 45,00 | | | |
| Beispiel 4 (1 %ig) [g] | | | | 15,00 | 30,00 | 45,00 |
| Polym.-Dauer [Std.] | 15,00 | 15,00 | 15,00 | 15,00 | 15,00 | 15,00 |
| Polym.-Temp. [°C] | 70,00 | 70,00 | 70,00 | 70,00 | 70,00 | 70,00 |
| B [g] | 568,84 | 567,95 | 570,44 | 564,83 | 567,20 | 572,46 |
| T [g] | 369,34 | 368,56 | 371,09 | 365,66 | 367,50 | 373,02 |
| Ausfall, getr. [g] | 1,40 | 0,09 | 0,22 | 0,07 | 0,20 | 0,20 |
| Feststoff [%] | 11,98 | 14,17 | 13,30 | 13,79 | 12,87 | 14,83 |
| pH-Wert | 8,10 | 8,10 | 8,10 | 8,50 | 8,60 | 8,40 |
| el. Leitfähigk. [mS] | 0,13 | 0,18 | 0,23 | 0,07 | 0,09 | 0,16 |
| Photometr. Messung | | | | | | |
| Wl=500 nm [nm] | 368,00 | 184,00 | 155,00 | 334,00 | 230,00 | 129,00 |
| Wl=600 nm [nm] | 339,00 | 176,00 | 150,00 | 315,00 | 219,00 | 124,00 |
| Wl=700 nm [nm] | 335,00 | 172,00 | 147,00 | 309,00 | 214,00 | 121,00 |

### Beispiel 19: Polymerisationsversuche mit Styrol

| | A | B | C | D | E | F |
|---|---|---|---|---|---|---|
| E-Wasser. ausgek.[g] | 153,32 | 136,64 | 119,96 | 153,44 | 136,88 | 120,32 |
| Natronlauge 0,1 n [ml] | 1,84 | 3,67 | 5,51 | 1,72 | 3,44 | 5,15 |
| Styrol [g] | 30,00 | 30,00 | 30,00 | 30,00 | 30,00 | 30,00 |
| Beispiel 5 (1 %ig) [g] | 15,00 | 30,00 | 45,00 | | | |
| Beispiel 6 (1 %ig) [g] | | | | 15,00 | 30,00 | 45,00 |
| Polym.-Dauer [Std.] | 15,00 | 15,00 | 15,00 | 15,00 | 15,00 | 15,00 |
| Polym.-Temp. [°C] | 70,00 | 70,00 | 70,00 | 70,00 | 70,00 | 70,00 |
| B [g] | 563,56 | 570,72 | 567,00 | 566,81 | 571,05 | 567,85 |
| T [g] | 364,19 | 371,20 | 367,20 | 366,44 | 371,39 | 368,28 |
| Ausfall, getr. [g] | 0,52 | 0,17 | 0,14 | 4,70 | 1,10 | 0,46 |
| Feststoff [%] | 14,31 | 14,67 | 14,85 | 9,06 | 13,97 | 14,67 |
| pH-Wert | 6,90 | 7,40 | 7,10 | 7,40 | 7,60 | 7,80 |
| el. Leitfähigk. [mS] | 0,12 | 0,20 | 0,28 | 0,10 | 0,15 | 0,20 |
| Photometr. Messung | | | | | | |
| Wl=500 nm [nm] | 218,00 | 134,00 | 130,00 | 534,00 | 347,00 | 187,00 |
| Wl=600 nm [nm] | 130,00 | 123,00 | 475,00 | 327,00 | 170,00 | 206,00 |
| Wl=700 nm [nm] | 127,00 | 119,00 | 439,00 | 313,00 | 163,00 | 201,00 |

### Beispiel 20: Polymerisationsversuche mit Styrol

| | A | B | C | D | E | F |
|---|---|---|---|---|---|---|
| E-Wasser. ausgek.[g] | 153,54 | 137,08 | 120,63 | 153,03 | 136,06 | 119,09 |
| Natronlauge 0,1 n [ml] | 1,61 | 3,23 | 4,84 | 2,13 | 4,26 | 6,38 |
| Styrol [g] | 30,00 | 30,00 | 30,00 | 30,00 | 30,00 | 30,00 |
| Beispiel 7 (1 %ig) [g] | 15,00 | 30,00 | 45,00 | | | |
| Beispiel 8 (1 %ig) [g] | | | | 15,00 | 30,00 | 45,00 |
| Polym.-Dauer [Std.] | 15,00 | 15,00 | 15,00 | 15,00 | 15,00 | 15,00 |
| Polym.-Temp. [°C] | 70,00 | 70,00 | 70,00 | 70,00 | 70,00 | 70,00 |
| B [g] | 512,10 | 565,75 | 567,27 | 567,26 | 571,62 | 569,10 |
| T [g] | 372,32 | 365,70 | 366,98 | 367,71 | 371,57 | 368,77 |
| Ausfall feucht [g] | 0,50 | 0,80 | 0,90 | 0,40 | 5,60 | 1,40 |
| Ausfall, getr. [g] | 0,31 | 0,70 | 0,50 | 0,37 | 1,80 | 0,90 |
| Feststoff [%] | 13,84 | 14,66 | 14,87 | 13,65 | 14,05 | 14,61 |
| Theorie [%] | 15,10 | 15,19 | 15,29 | 15,11 | 15,21 | 15,32 |
| pH-Wert | 7,50 | 7,60 | 7,40 | 6,60 | 6,30 | 6,60 |
| el. Leitfähigk. [mS] | 0,12 | 0,19 | 0,27 | 0,21 | 0,39 | 0,55 |
| Photometr. Messung | | | | | | |
| Wl=500 nm [nm] | 278,00 | 164,00 | 108,00 | 244,00 | 188,00 | 157,00 |
| Wl=600 nm [nm] | 268,00 | 159,00 | 102,00 | 224,00 | 178,00 | 151,00 |
| Wl=700 nm [nm] | 262,00 | 158,00 | 96,00 | 214,00 | 172,00 | 147,00 |

### Beispiel 21: Polymerisationsversuche mit Styrol

| | A | B | C | D | E | F |
|---|---|---|---|---|---|---|
| E-Wasser. ausgek.[g] | 152,63 | 135,26 | 117,89 | 152,28 | 134,56 | 117,89 |
| Natronlauge 0,1 n [ml] | 2,53 | 5,06 | 7,59 | 2,88 | 5,76 | 8,65 |
| Styrol [g] | 30,00 | 30,00 | 30,00 | 30,00 | 30,00 | 30,00 |
| Beispiel 9 (1 %ig) [g] | 15,00 | 30,00 | 45,00 | | | |
| Beispiel 10 (1 %ig) [g] | | | | 15,00 | 30,00 | 45,00 |
| Polym.-Dauer [Std.] | 15,00 | 15,00 | 15,00 | 15,00 | 15,00 | 15,00 |
| Polym.-Temp. [°C] | 70,00 | 70,00 | 70,00 | 70,00 | 70,00 | 70,00 |
| B [g] | 569,07 | 568,52 | 572,42 | 565,48 | 567,42 | 574,41 |
| T [g] | 369,36 | 368,59 | 372,12 | 365,70 | 367,40 | 372,90 |
| Ausfall feucht [g] | 21,70 | 25,60 | 25,20 | 24,00 | 31,80 | 25,90 |
| Ausfall, getr. [g] | 3,70 | 4,70 | 5,20 | 3,50 | 5,20 | 5,00 |
| Feststoff [%] | 0,36 | 0,34 | 0,40 | 0,47 | 0,31 | 0,65 |

Der Einsatz dieser Verbindungen führte wegen nicht ausreichender Emulgatorwirkung zu zweiphasigen Gemischen, die klebrige und schmierige Dispersionen zur Folge hatten.

### Beispiel 22: Polymerisationsversuche mit Styrol

| | A | B | C | D | E | F |
|---|---|---|---|---|---|---|
| E-Wasser. ausgek.[g] | 152,83 | 135,67 | 118,50 | 151,63 | 135,67 | 118,50 |
| Natronlauge 0,1 n [ml] | 2,33 | 4,65 | 6,98 | 3,53 | 7,07 | 10,60 |
| Styrol [g] | 30,00 | 30,00 | 30,00 | 30,00 | 30,00 | 30,00 |
| Beispiel 11 (1 %ig) [g] | 15,00 | 30,00 | 45,00 | | | |
| Beispiel 12 (1 %ig) [g] | | | | 15,00 | 30,00 | 45,00 |
| Polym.-Dauer [Std.] | 15,00 | 15,00 | 15,00 | 15,00 | 15,00 | 15,00 |
| Polym.-Temp. [°C] | 70,00 | 70,00 | 70,00 | 70,00 | 70,00 | 70,00 |
| B [g] | 563,30 | 571,10 | 567,60 | 566,07 | 573,82 | 572,83 |
| T [g] | 364,11 | 371,08 | 367,14 | 365,93 | 371.29 | 368,20 |
| Ausfall feucht [g] | 20,00 | 22,60 | 22,00 | 26,00 | 27,90 | 33,30 |
| Ausfall, getr. [g] | 4,50 | 6,50 | 7,60 | 1,50 | 2,30 | 3,30 |
| Feststoff [%] | 0,20 | 0,35 | 0,27 | 0,03 | 0,08 | 0,11 |

Der Einsatz dieser Verbindungen führte wegen nicht ausreichender Emulgatorwirkung zu zweiphasigen Gemischen, die klebrige und schmierige Dispersionen zur Folge hatten.

### Beispiel 23: Polymerisationsversuche mit Styrol

| | A | B | C |
|---|---|---|---|
| E-Wasser. ausgek.[g] | 152,43 | 134,85 | 117,28 |
| Natronlauge 0,1 n [ml] | 2,73 | 5,47 | 8,21 |
| Styrol [g] | 30,00 | 30,00 | 30,00 |
| Beispiel 13 (1 %ig) [g] | 15,00 | 30,00 | 45,00 |
| Polym.-Dauer [Std.] | 15,00 | 15,00 | 15,00 |
| Polym.-Temp. [°C] | 70,00 | 70,00 | 70,00 |
| B [g] | 568,00 | 571,26 | 570,76 |
| T [g] | 368,15 | 371,01 | 370,40 |
| Ausfall feucht [g] | 26,90 | 24,20 | 24,50 |
| Ausfall, getr. [g] | 5,80 | 5,70 | 3,30 |
| Feststoff [%] | 0,13 | 0,33 | 0,11 |

Der Einsatz dieser Verbindungen führte wegen nicht ausreichender Emulgatorwirkung zu zweiphasigen Gemischen, die Klebrige und schmierige Dispersionen zur Folge hatten.

### Beispiel 24: Polymerisationsversuche mit Styrol

| | A | B | C | D | E | F |
|---|---|---|---|---|---|---|
| E-Wasser. ausgek.[g] | 155 | 140 | 125 | 155 | 140 | 125 |
| Styrol [g] | 30 | 30 | 30 | 30 | 30 | 30 |
| Beispiel 14 | 15 | 30 | 45 | | | |
| Beispiel 15 | | | | 15 | 30 | 45 |
| Polym.-Dauer [h] | 15 | 15 | 15 | 15 | 15 | 15 |
| Temp. [°C] | 70 | 70 | 70 | 70 | 70 | 70 |
| Brutto | 568,07 | 565,58 | 566,86 | 567,24 | 567,9 | 567,1 |
| Tara | 368,3 | 365,59 | 366,9 | 367,39 | 367,48 | 367,11 |
| Feststoff | 14,23 | 14,49 | 14,79 | 14,32 | 14,75 | 14,64 |
| Latexgewicht | 195,08 | 193,43 | 194,28 | 195,48 | 196,28 | 195,47 |
| Ausfall feucht | 1,04 | 1,09 | 0,92 | 0,78 | 0,62 | 0,9 |
| Ausfall trocken | 0,8 | 0,58 | 0,36 | 0,51 | 0,37 | 0,53 |

Diese Beispiele zeigen, daß eine Mindestzahl von C-Atomen erforderlich ist, um eine ausreichende Wirkung zu erzielen.

### Beispiel 25: Vergleichsversuche

| | A | B | C | D | E | F | G | H | I |
|---|---|---|---|---|---|---|---|---|---|
| Entionisiertes Wasser [g] | 159,1 | 148,3 | 105,1 | 163,6 | 152,80 | 109,6 | 163,6 | 152,8 | 109,6 |
| Styrol [g] | 100,0 | 100,0 | 100,0 | 42,7 | 42,70 | 42,7 | 42,7 | 42,7 | 42,7 |
| n-Butylacrylat [g] | 57,3 | 57,3 | 57,3 | 57,3 | 57,3 | 57,3 | 57,3 | 57,3 | 57,3 |
| Alkalialkanmonosulfonat mit ca. 15 C-Atomen | 36,0 | 48,0 | 96,0 | 28,5 | 38,00 | 76,0 | | | |
| Alkalialkandisulfonat mit ca. 15 C-Atomen | | | | | | | 28,5 | 38,0 | 76,0 |
| Azodiisobuttersäureamidin 10 %ig [g] | 5,0 | 5,0 | 5,0 | 7,5 | 10,00 | 10,0 | 20,0 | 20,0 | 7,5 |
| Temperatur[°C] | 60,0 | 60,0 | 60,0 | 60,0 | 60,00 | 60,0 | 60,0 | 60,0 | 60,0 |
| Dauer [Stunden] | 7,0 | 7,0 | 7,0 | 7,0 | 7,00 | 7,0 | 7,0 | 7,0 | 7,0 |
| Feststoffgehalt [%] | 31,9 | 33,8 | 30,8 | 33,8 | 34,80 | 34,6 | 34,5 | 31,2 | 31,4 |
| Elektrische Leitfähigkeit [mS] | 2,5 | 2,9 | 4,6 | 3,5 | 4,50 | 4,8 | 6,8 | 10,8 | 7,8 |
| Latexteilchendurchmesser [nm] | 108,0 | 107,0 | 95,0 | 94,0 | 90,00 | 153,0 | 79,0 | 150,0 | 220,0 |
| Schaumrückbildungszeit [sec] | >300,0 | >300,0 | >300,0 | >300,0 | >300,0 | >300,0 | >300,0 | >300,0 | >300,0 |

Die elektrische Leitfähigkeit, die als Maß für die chemische Belastung des Abwassers verwendet werden kann, ist in den erfindungsgemäßen Beispielen um etwa den Faktor 10 kleiner als im Vergleich, wodurch die Vorteile der neuen Initiatoren deutlich bewiesen werden.

### Beispiel 26: Vergleichsbeispiel

| | A | B | C | D | E |
|---|---|---|---|---|---|
| E-Wasser, ausgek. | 204,59 | 201,31 | 194,75 | 181,64 | 168,52 |
| Styrol [g] | 57 | 57 | 57 | 57 | 57 |
| Methacrylsäure [g] | 0,285 | 0,571 | 1,141 | 2,282 | 3,423 |
| Kalilauge, 1 n [g] | 3,46 | 6,93 | 13,86 | 27,71 | 41,57 |
| Kaliumpersulfat-Lös. 1 %ig [g] | 11,4 | 11,4 | 11,4 | 11,4 | 11,4 |
| Sodalösung 1 %ig [g] | 8,94 | 8,94 | 8,94 | 8,94 | 8,94 |
| Temp. [°C] | 70 | 70 | 70 | 70 | 70 |
| Dauer [h] | 15 | 15 | 15 | 15 | 15 |
| Feststoffgehalt [%] | 4,08 | 15,62 | 18,27 | 18,28 | 17,98 |
| El. Leitfähgkeit [mS] | 1,98 | 3,00 | 4,77 | 8,67 | 12,47 |
| Latexteilchendurchmesser [nm] | >1000 | 790 | 672 | 483 | 396 |

### Beispiel 27: Vergleichsbeispiel

| | A | B | C | D | E |
|---|---|---|---|---|---|
| E-Wasser, ausgek. | 203,95 | 200,02 | 192,20 | 176,53 | 160,87 |
| Styrol [g] | 57 | 57 | 57 | 57 | 57 |
| Acrylsäure [g] | 0,285 | 0,571 | 1,141 | 2,282 | 3,423 |
| Kalilauge, 1 n [g] | 4,13 | 8,28 | 16,55 | 33,11 | 49,66 |
| Kaliumpersulfat-Lös. 1 %ig [g] | 11,4 | 11,4 | 11,4 | 11,4 | 11,4 |
| Sodalösung 1 %ig [g] | 8,94 | 8,94 | 8,94 | 8,94 | 8,94 |
| Temp. [°C] | 70 | 70 | 70 | 70 | 70 |
| Dauer [h] | 15 | 15 | 15 | 15 | 15 |
| Feststoffgehalt [%] | 16,73 | 8,41 | 17,37 | 16,62 | 18,36 |
| El. Leitfähgkeit [mS] | 2,16 | 3,50 | 5.70 | 10,25 | 14,20 |
| Latexteilchendurchmesser [nm] | 767 | >1000 | 734 | 446 | 437 |

Die elektrische Leitfähigkeit, die als Maß für die chemische Belastung des Abwassers verwendet werden kann, ist auch in diesen beiden Vergleichsbeispielen um etwa den Faktor 10 größer als in den erfindungsgemäßen Beispielen, wodurch die Vorteile der neuen Initiatoren deutlich bewiesen werden.

## Patentansprüche

1. Verwendung der Peroxide der Formel (I) 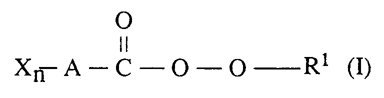 worin bedeuten
R¹ Alkyl-, Cycloalkyl- und Arylreste mit 4 bis 20 Kohlenstoffatomen,
X die Reste -COOH und -SO₃H sowie deren Alkali-, Erdalkali- oder Ammoniumsalze,
n eine ganze Zahl im Bereich von 1 bis 6,
A einen Alkylenrest mit 6 bis 40 Kohlenstoffatomen,
zur Emulsionspolymerisation olefinisch ungesättigter Monomerer.

2. Verwendung gemaß Anspruch 1, dadurch gekennzeichnet, daß es sich bei dem Rest A in Formel (I) um einen Rest der Formel (II) handelt 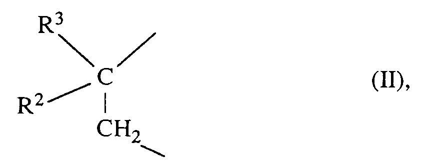 worin
die Reste R² und R³ für H, C₁-C₄₀-Alkyl, C₃-C₄₀-Cycloalkyl, Aryl, Aralkyl oder Halogen stehen, mit der Einschränkung, daß die Summe der Kohlenstoffatome beider Reste größer als 16 ist.

3. Verwendung gemaß Anspruch 1, dadurch gekennzeichnet, daß man 0,05 bis 10 Gew.-%, bezogen auf Monomere, der Peroxide einsetzt

4. Peroxide der Formel (I) 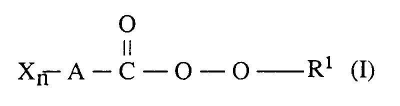 worin bedeuten
R¹ Alkyl-, Cycloalkyl- und Arylreste mit 4 bis 20 Kohlenstoffatomen,
X die Reste -COOH und -SO₃H sowie deren Alkali-, Erdalkali- oder Ammoniumsalze,
n eine ganze Zahl im Bereich von 1 bis 6 und
A einen Rest der Formel (II) bedeutet, 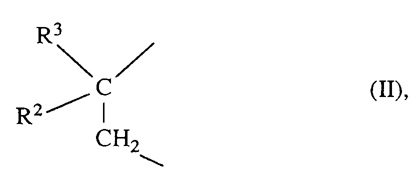
worin
die Reste R² und R³ für H, C₁-C₄₀-Alkyl, C₃-C₄₀-Cycloalkyl, Aryl, Aralkyl oder Halogen stehen, mit der Einschränkung, daß die Summe der Kohlenstoffatome beider Reste größer als 16 ist.

## Claims

1. Use of peroxides of the formula (I) 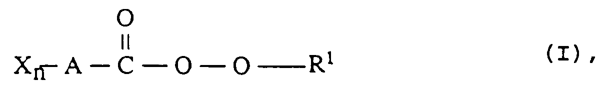 wherein
R¹ denotes alkyl, cycloalkyl and aryl radicals having 4 to 20 carbon atoms,
X denotes the radicals -COOH and -SO₃H and alkali metal, alkaline earth metal or ammonium salts thereof,
n denotes an integer in the range from 1 to 6, and
A denotes an alkylene radical having 6 to 40 carbon atoms,
for the emulsion polymerization of olefinically unsaturated monomers.

2. Use according to Claim 1, characterized in that the radical A in formula (I) is a radical of the formula (II) 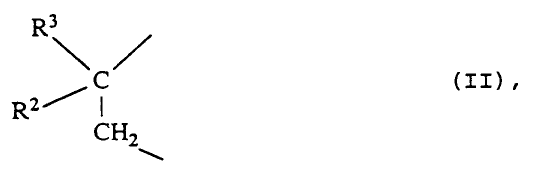 wherein
the radicals R² and R³ represent H, C₁-C₄₀-alkyl, C₃-C₄₀-cycloalkyl, aryl, aralkyl or halogen, with the limitation that the sum of the carbon atoms in the two radicals is greater than 16.

3. Use according to Claim 1, characterized in that from 0.05 to 10 % by weight, based on monomers, of the peroxides are employed.

4. Peroxides of the formula (I) 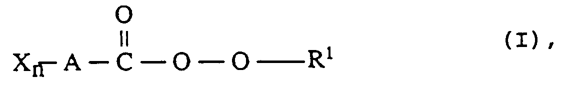 wherein
R¹ denotes alkyl, cycloalkyl and aryl radicals having 4 to 20 carbon atoms,
X denotes the radicals -COOH and -SO₃H and alkali metal, alkaline earth metal or ammonium salts thereof,
n denotes an integer in the range from 1 to 6, and
A denotes a radical of the formula (II) 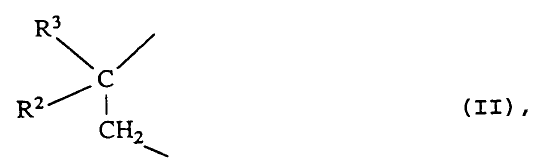
wherein
the radicals R² and R³ represent H, C₁-C₄₀-alkyl, C₃-C₄₀-cycloalkyl, aryl, aralkyl or halogen, with the limitation that the sum of the carbon atoms in the two radicals is greater than 16.

## Revendications

1. Utilisation des peroxydes de formule (I) 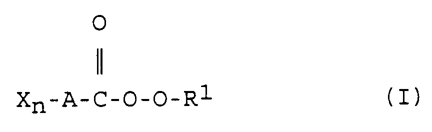 dans laquelle
R¹ représente un radical alkyle, un radical cycloalkyle et un radical aryle contenant de 4 à 20 atomes de carbone,
X représente les radicaux -COOH et -SO₃H, ainsi que leurs sels de métaux alcalins, de métaux alcalino-terreux ou d'ammonium,
n représente un nombre entier dans le domaine de 1 à 6,
A représente un radical alkylène contenant de 6 à 40 atomes de carbone,
pour la polymérisation en émulsion de monomères à insaturation oléfinique.

2. Utilisation selon la revendication 1, caractérisée en ce que, en ce qui concerne le radical A dans la formule (I), il s'agit d'un radical de formule (II) 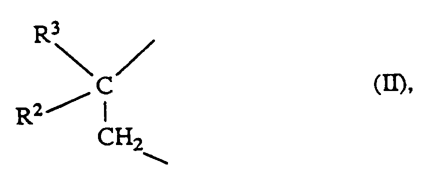 dans laquelle
les radicaux R² et R³ représentent H, un groupe alkyle en C₁-C₄₀, un groupe cycloalkyle en C₃-C₄₀, un groupe aryle, un groupe aralkyle ou un atome d'halogène, avec cette restriction que la somme des atomes de carbone des deux radicaux est supérieure à 16.

3. Utilisation selon la revendication 1, caractérisée en ce qu'on met en oeuvre de 0,05 à 10% en poids, rapportés aux monomères, des peroxydes.

4. Peroxydes de formule (I) 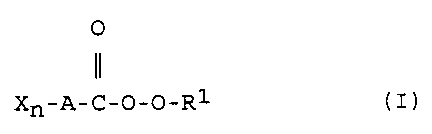 dans laquelle
R¹ représente un radical alkyle, un radical cycloalkyle et un radical aryle contenant de 4 à 20 atomes de carbone,
X représente les radicaux -COOH et -SO₃H, ainsi que leurs sels de métaux alcalins, de métaux alcalino-terreux ou d'ammonium,
n représente un nombre entier dans le domaine de 1 à 6,
A représente un radical de formule (II) 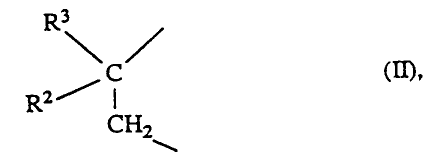
dans laquelle
les radicaux R² et R³ représentent H, un groupe alkyle en C₁-C₄₀, un groupe cycloalkyle en C₃-C₄₀, un groupe aryle, un groupe aralkyle ou un atome d'halogène, avec cette restriction que la somme des atomes de carbone des deux radicaux est supérieure à 16.
